# EUROPÄISCHE PATENTSCHRIFT

(11) **EP 1 275 638 B1**
(45) Veröffentlichungstag und Bekanntmachung des Hinweises auf die Patenterteilung: **14.06.2006**
(21) Anmeldenummer: 02013275.9
(22) Anmeldetag: 18.06.2002
(51) Int. Cl.: C07C 255/21, A61K 31/275, A61K 45/06, A61P 37/06

(54) **Kombinationspräparat zur Therapie von immunologischen Erkrankungen**
Combination therapy for the treatment of immunological disorders
Thérapie combineé pour le traitement des maladies immunologiques

(30) Priorität: 06.07.2001 DE 10132308
(43) Veröffentlichungstag der Anmeldung: 15.01.2003
(73) Patentinhaber: Sanofi-Aventis Deutschland GmbH, 65929 Frankfurt am Main (DE)
(72) Erfinder: Lindner, Juergen, Dr., 60318 Frankfurt (DE)

(56) Entgegenhaltungen:
- EP-A- 0 754 459
- EP-A- 0 945 465
- WO-A-98/24463
- WO-A-98/24477
- JP-A- 6 298 654
- US-A- 5 747 034
- US-B1- 6 299 878

## Beschreibung

Gegenstand der Erfindung ist ein pharmazeutisches Kombinationspräparat, mit dem eine überschießende, schädigende Immunreaktion sowie degenerative Prozesse durch den Aufbau einer regulatorischen Immunantwort therapiert werden können.

Überschießende, schädigende Immunreaktionen sind Immunreaktionen gegen körpereigene und/oder körperfremde Stoffe, die den Organismus mehr schädigen als nützen. Überschießende, schädigende Immunreaktionen gegen körperfremde Substanzen können allergische Reaktionen, Transplantatabstoßungen oder lmmunreaktionen gegen gentechnisch veränderte Zellen oder deren Produkte wie Faktor VIII oder Insulin sein. Beispiele für überschießende, schädigende Reaktionen gegen körpereigene Stoffe sind Autoimmunerkrankungen wie Multiple Sklerose, rheumatoide Arthritis, Pemphigus vulgaris und Thyreoiditis Hashimoto. Bei einigen Erkrankungen wie Colitis ulcerosa, Morbus Crohn, Psoriasis und Arteriosklerose treten sowohl gegen körpereigene als auch gegen körperfremde Substanzen überschießende Immünreaktionen auf, die den Organismus mehr schädigen als ihm nützen. Bei den sog. Kontaktallergien treten Immunreaktionen gegen körperfremde Antigene und/oder durch chemische Verbindungen veränderte körpereigene Strukturen auf, zum Beispiel bei den Kontaktallergien gegen Nickel oder Chrom.

Unter degenerativen Prozessen versteht man Erkrankungen, die sich durch ein Ungleichgewicht von Gewebeaufbau und Gewebeabbau auszeichnen. Hierzu gehören beispielsweise Arthrose, Demenserkrankungen und Colitis ulcerosa.

Unter einer antigen-spezifischen Effektoraktivität des Immunsystems versteht man die verstärkte Immunreaktion des Immunsystems gegen ein spezifisches, meist bereits bekanntes Antigen. Dadurch werden heftige Reaktionen ausgelöst, die zu einer Zerstörung von Zellen führen oder eine Immunreaktion gegen das erkannte Antigen erheblich steigern.

Unter einer antigen-spezifischen Suppressoraktivität versteht man eine Reaktion des Immunsystems, bei der die Suppressorzellen mit einem meist bekannten spezifischen Antigen so reagieren, dass die Immunreaktion gegen die erkannte Struktur herunterreguliert wird. Überwiegt die Suppressoraktivität die Effektoraktivität, so sind Reaktionen des Immunsystems gegen die von den Suppressorzellen erkannten Strukturen nicht mehr möglich.

Normalerweise reagiert das Immunsystem des Organismus auf das Antigen mit einer regulatorischen Immunantwort, bei der eine antigen-spezifische, suppressorische Aktivität gegenüber der proinflammatorischen oder cytoxischen Aktivität des Immunsystems überwiegt. Das so entstehende Gleichgewicht zwischen suppressorischer und proinflammatorischer Aktivität des Immunsystems kann nun allerdings entscheidend gestört werden, wenn eine zusätzliche entzündliche Reaktion auftritt. Diese verschiebt dann das Gleichgewicht von der antigen-spezifischen Suppressoraktivität hin zu einer antigen-spezifischen proinflammatorischen oder cytotoxischen Effektoraktivität. Sobald die Entzündungsreaktion eliminiert ist, verschiebt sich durch die regulatorische Immunantwort das Gleichgewicht zwischen Suppressoraktivität und Effektoraktivität wieder zu einem Übergewicht der Suppressoraktivität.

Die bisher übliche Therapie von Erkrankungen, die durch überschießende, schädigende Immunreaktionen verursacht werden, erfolgt in der Regel symptomatisch. Dabei werden die Immunreaktion und deren unerwünschte Begleiterscheinungen relativ unspezifisch unterdrückt. Immunsuppressive Medikamente wie Cyclosporin A, Korticoide oder Cyclophosphamid unterdrücken derartige Erkrankungen zwar sehr effektiv, sind jedoch mit erheblichen Nebenwirkungen verbunden. Sie haben außerdem den Nachteil, dass nach dem Absetzen dieser Medikamente die Erkrankung wieder auftritt und es dabei im Rahmen des Erkrankungsschubs sehr oft zu irreparablen Schädigungen kommt. Die bisher existierenden Behandlungsmethoden für Erkrankungen, die durch überschießende, schädigende Reaktionen des Immunsystems gegen bestimmte Antigene hervorgerufen werden, sind deshalb entweder wenig wirksam oder mit erheblichen Nebenwirkungen verbunden. Es besteht deshalb ein großer Bedarf an Arzneizubereitungen, die überschießende, schädigende Reaktionen des Immunsystems verhindern und dabei gleichzeitig wenig Nebenwirkungen aufweisen. Die Behandlung mit einem derartigen Arzneipräparat darf allerdings zu keiner Beeinträchtigung des Immunsystems führen, da unter bestimmten pathologischen Bedingungen Autoimmunreaktionen physiologisch sehr sinnvoll sind, zum Beispiel bei einer Autoimmunreaktion gegen körpereigene Tumorzellen oder mit Viren infizierten Zellen. Allerdings muss diese Autoimmunreaktion nach der Zerstörung der Tumorzellen oder der mit Viren infizierten Zellen selbständig wieder zum Stillstand kommen und darf kein gesundes, körpereigenes Gewebe zerstören. Da von immunsuppressiven Zellen bekannt ist, dass sie u.a. den immunsuppressiven Faktor TGFβ (Tissue Growth Factor β) produzieren, können diese Zellen auch die Regeneration von Gewebe stimulieren.

Es wurde nun ein pharmazeutisches Kombinationspräparat gefunden, das den Aufbau einer regulatorischen Immunantwort zur Therapie einer überschießenden, schädigenden Immunreaktion sowie eine Therapie von degenerativen Prozessen unter Vermeidung einer akut entzündlichen Reaktion ermöglicht, wenn es
a) mindestens einen Pyrimidinsynthese-Inhibitor aus der Gruppe Brequinar, Verbindung der Formeln I oder II 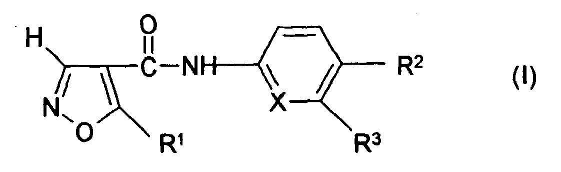 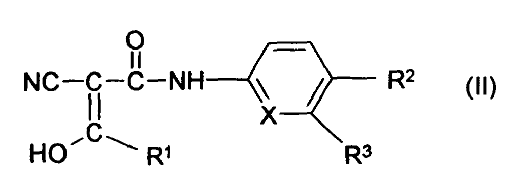 stereoisomene Formen von Verbindungen der Formel (I) oder (II) und ein physiologisch verträgliches Salz der Verbindung der Formel (II), in der die Substituenten folgende Bedeutung haben:
   - R¹ ist: a) -(C₁-C₄)-Alkyl.
   b) -(C₃-C₅)-Cycloalkyl,
   c) -(C₂-C₆)-Alkenyl, oder
   d) -(C₂-C₆)-Alkynyl;
   - R² ist: a) -CF₃,
   b) -O-CF₃,
   c) -S-CF₃,
   d) -OH,
   e) -NO₂,
   f) Halogen,
   g) Benzyl,
   h) Phenyl,
   i) -O-phenyl, welches unsubstituiert ist,
   j) -CN, oder
   k) -O-phenyl, das mono- oder polysubstituiert ist durch eine Gruppe ausgewählt aus
      1) (C₁-C₄)-Alkyl,
      2) Halogen,
      3) -O-CF₃ und
      4) -O-CH₃;
   - R³ ist: a) -(C₁-C₄)-Alkyl,
   b) Halogen, oder
   c) ein Wasserstoffatom; oder
   - X ist: a) eine CH-Gruppe
   b) ein Stickstoffatom.
   oder eine Mischung von Brequinar und Verbindungen der Formel (I) und (II) oder von Salzen der Verbindungen der Formel (II) und
b) mindestens ein Antigen aus der Gruppe natürliche oder künstlich veränderte Zellverbände, Zellen, Zellfragmente, Proteine, Proteinfragmente, Vorstufen von Antigenen oder andere Verbindungen, die antigen wirksam sind,
enthält.

Ganz besonders bevorzugt ist es für das erfindungsgemäße Kombinationspräparat, wenn als Pyrimidinsynthese -Inhibitor das N-(4-Trifluoromethylphenyl)-5-methylisoxazol-4-carboxamid der Formel (I) oder N-(4-Trifluoromethylphenyl)-2-cyano-5-hydroxycrotonamid, 2-Cyano-3-cyclopropyl-3-hydroxyacrylsäure (4-cyanophenyl)amid oder N-(4-Trifluoromethylphenyl)-2-cyano-3-hydroxyhept-2-en-6-yncarboxamid als Verbindungen der Formel (II) eingesetzt werden.

Verbindungen der Formeln I oder II können nach Verfahren hergestellt werden, die aus den europäischen Patentanmeldungen 484 223, 529 500, 538 783 und 551 230 sowie der US-Patentschrift 4 061 767 bekannt sind.

Ganz besonders geeignet ist Leflunomid, das bisher als Antirheumatikum eingesetzt wird. Es kann bei Verabreichung als Einzelsubstanz die Progression der rheumatoiden Arthritis reduzieren, aber nicht heilen. Soweit in einigen Veröffentlichungen über den Einsatz von Leflunomid bei tierexperimentellen Autoimmunkrankheiten berichtet wurde, dass eine Heilung von Autoimmunerkrankungen möglich sein soll, hat sich jedoch gezeigt, dass in den genannten Untersuchungen Leflunomid präventiv gegeben wurde und somit nur die Entstehung einer Autoimmunerkrankung verhindern und die Symptome einer Autoimmunerkrankung effektiv unterdrücken konnte und somit die Überlebensrate der Versuchstiere erhöhte. Die Tatsache, dass durch die Verabreichung von Leflunomid die Entwicklung einer Autoimmunerkrankung verhindert wurde, wurde als kurativer Therapieeffekt bezeichnet. Diese Art von "kurativem" Effekt hat jedoch nichts mit einer kurativen Therapie einer Autoimmunerkrankung zu tun, bei der nach dem Absetzen der Arzneimittelzugabe die Erkrankung nicht mehr auftritt. Die bisherigen Berichte über die Wirkung von Leflunomid bei Patienten mit rheumatoider Arthritis haben keinen Hinweis dafür gegeben, dass es nach dem Absetzen von Leflunomid zu keinem Erkrankungsschub mehr kommt und die Patienten langfristig geheilt sind. Das bisherige Wissen über den Wirkungsmechanismus von Leflunomid lässt sich dahingehend zusammenfassen, dass in vitro Leflunomid klassische immunsuppressorische Wirkungen zeigt (Hemmung der Pyrimidinbiosynthese, Hemmung der Tyrosinphosphorylierung und Inhibition des Nuklear Factor Kappa B Signaltransduktionsweges). In vivo vermittelt Leflunomid anscheinend seine Wirkung über Lymphozyten. Nur vereinzelt wird die Induktion des Phänomens einer antigen-spezifischen Toleranz durch Leflunomid beobachtet, jedoch tritt diese nur sporadisch auf und ist daher für eine gezielte klinische Anwendung nicht geeignet. Sowohl in der klinischen als auch in der tierexperimentellen Untersuchung hat sich gezeigt, dass Leflunomid die Infektionsanfälligkeit nicht vergrößert.

Da eine Proliferation von Zellen des Immunsystems nur solange erfolgt, bis die zugrundeliegende Immunreaktion gestoppt ist, erfolgt bei der normalen klinischen Anwendung von Leflunomid nur solange eine Expansion von antigen-spezifischen suppressorischen Zellen des Immunsystems bis die unerwünschte Immunantwort zum Stillstand kommt. Eine relativ selektive Expansion der immunsuppressiven Zellen unter Leflunomid erfolgt nur solange, bis die direkt immunsuppressive Wirkung von Leflunomid und die suppressive Wirkung der neugebildeten Suppressor zellen gerade die unerwünschte Immunreaktion zum Stillstand bringen. Wird in dieser Situation dann Leflunomid abgesetzt, so reicht die verbleibende Wirkung der suppressorisch wirkenden Zellen zur Kontrolle der unerwünschten Immunreaktion nicht aus.

Daher kommt es bei dem im Augenblick üblichen Einsatz von Leflunomid in der Klinik nicht zu einem kurativen Therapieergebnis, bei dem Leflunomid abgesetzt werden kann, ohne dass die zugrundeliegende immunologische Erkrankung wieder ausbricht.

Das erfindungsgemäße Kombinationspräparat enthält nun als weiteren wesentlichen Bestandteil ein oder mehrere Antigene, welche an der unerwünschten Immunreaktion bzw. an dem degenerativen Prozess beteiligt sind. Als Antigen enthält das erfindungsgemäße Kombinationspräparat natürliche oder künstlich veränderte Zellverbände, Zellen, Zellfragmente, Proteine, Proteinfragmente oder andere Verbindungen, die antigen wirksam sind. Durch die gleichzeitige Gabe des Antigens, gegen das die regulatorische Immunantwort aufgebaut werden soll, und einen Pyrimidinsynthese-Inhibitor wie Leflunomid werden die rasch proliferierenden, proinflammatorischen/zytotoxischen Effektorzellen des Immunsystems in ihrer Proliferation gehemmt und bleiben in der späten G1-Phase stehen. Im Gegensatz zu den Effektorzellen werden wahrscheinlich die langsamer proliferierenden Suppressorzellen durch Leflunomid in ihrer Proliferation nicht gestört. Hierdurch kommt es nach einiger Zeit zu einem Überwiegen der Aktivität der Suppressorzellen über die Effektorzellen.

Dadurch, dass das die unerwünschte Immunreaktion bzw. das den degenerativen Prozess verursachende/beteiligte Antigen zusätzlich zu Leflunomid verabreicht wird, wird die Proliferation von antigen-spezifischen suppressiven Zellen des Immunsystems auch dann noch aufrechterhalten, wenn die zugrundeliegende unerwünschte Immunreaktion schon beendet ist. Daher kann durch die kombinierte Gabe des verursachenden Antigens und Leflunomid die antigen-spezifische suppressorisch wirkende Zellpopulation soweit vermehrt werden, dass ein gewisser Überschuss an suppressorischer Aktivität auch nach dem Absetzen der Leflunomidgabe übrigbleibt und ein Wiederausbrechen der unerwünschten schädlichen lmmunreaktion verhindert wird bzw. den Regenerationsprozess fördernde Faktoren weiterhin freigesetzt werden.

Die durch das erfindungsgemäße Kombinationspräparat hervorgerufene Reaktionslage des Immunsystems mit einem Überwiegen der antigen-spezifischen Suppressor-aktivität kann durch unspezifische Entzündungsreaktionen kurzzeitig zusammenbrechen, baut sich jedoch nach dem Verschwinden der unspezifischen Entzündungsreaktion ohne weitere medikamentöse Eingriffe wieder auf. Es wurde jedoch gefunden, dass die Erhöhung der Aktivität antigen-spezifischer, immunsuppressiver Zellen nur dann gelingt, wenn akut entzündliche Prozesse vermieden werden. Deshalb ist es für das erfindungsgemäße Kombinationspräparat ggf. erforderlich, dass es einen eine akute entzündliche Reaktion unterdrückenden Wirkstoff enthält. Zum Aufbau einer regulatorischen Immunantwort müssen deshalb akut entzündliche Reaktionen zum Beispiel durch Gabe von Steroiden oder anderen anti-entzündlich wirkenden Substanzen kontrolliert werden. Hierzu gehören Antihistaminika, Mastzellstabilisatoren wie Cromoglycinsäure, Substanzen, die direkt oder indirekt wie anti-entzündliche Mediatoren wirken, zum Beispiel TGFβ, und Prostaglandin E2 oder Substanzen, die die toxischen Mediatoren, die im Rahmen eines Entzündungsprozesses freigesetzt werden, unterdrücken oder antagonisieren. Allerdings darf durch den Einsatz dieser entzündungshemmenden Wirkstoffe die Immunreaktion nicht vollständig unterdrückt werden, da sonst kein Aufbau einer regulatorischen Immunreaktion möglich ist.

Eine antigen-spezifische, regulatorische Immunantwort zur kurativen Behandlung von überschießenden, schädigenden Immunreaktionen und zur Behandlung von degenerativen Prozessen wird also erfindungsgemäß dadurch aufgebaut, dass zeitlich befristet gleichzeitig oder zeitlich versetzt immunmodulierende Wirkstoffe und dasjenige oder diejenigen Antigene gegeben werden, gegen die die unerwünschte Immunreaktion abläuft bzw. Antigene, die bei degenerativen Prozessen eine Rolle spielen. Wichtig ist, dass während des Aufbaus der antigen-spezifischen regulatorischen Immunreaktion keine akut entzündlichen Reaktionen ablaufen, da sich sonst keine regulatorische Immunantwort aufbauen kann. Unter Beibehaltung der Verabreichung der immunmodulatorischen Substanzen und der regelmäßigen Verabreichung des/der Antigene hat es sich als vorteilhaft erwiesen, stufenweise die Verabreichung von anti-entzündlichen Wirkstoffen zu reduzieren, zum Beispiel durch das langsame Ausschleichen der Steroidmedikation zur Vermeidung von akut-entzündlichen Reaktionen.

Sobald sämtliche Maßnahmen zur Vermeidung von akut-entzündlichen Reaktionen während der Verabreichung des Kombinationspräparats abgesetzt werden können, ist es ratsam, die medikamentöse Behandlung noch für mehrere Wochen fortzusetzen, damit sich eine stabile regulatorische Immunantwort ausbilden kann, die auch nach dem Absetzen sämtlicher therapeutischer Maßnahmen das Wiederauftreten der unerwünschten schädigenden Immunantwort verhindert. Bei über lange Zeit bestehenden progressiven unerwünschten Immunreaktionen, zum Beispiel schnell verlaufender Multipler Sklerose, kann es vorteilhaft sein, diesen Zeitraum länger zu gestalten (zum Beispiel 6 Monate), damit sich eine genügende Anzahl von suppressorischen Zellen des Immunsystems bilden kann, die die Effektorzellen auch unter leicht entzündlichen Bedingungen, zum Beispiel bei einem grippalen Effekt, noch unter Kontrolle halten.

Nach diesem Zeitraum der Verabreichung des Kombinationspräparates von Antigen und Proteinbiosynthese-Inhibitor kann es vorteilhaft sein, die Antigene noch über einen kurzen Zeitraum als Monotherapie, also ohne Proteinbiosynthese-Inhibitor, zu verabreichen, bevor sämtliche therapeutischen Maßnahmen beendet werden können.

Bei den Maßnahmen, die eine akut-entzündliche Reaktion während des Aufbaues der regulatorischen Immunantwort vermeiden, kann es sich um Therapieprinzipien handeln, die direkt an den Pyrimidinsynthese-Inhibitor, das Antigen oder eine Vorstufe des Antigens physikalisch oder chemisch gekoppelt sind oder getrennt davon verabreicht werden. Bei den Therapieprinzipien kann es sich zum Beispiel um Substanzen mit Steroidwirkung (zum Beispiel Fluocortolon oder Dexamethason), nicht steroidale Antiphlogistika (zum Beispiel Diclofenac, Indometacin, Ibuprofen, allgemein Hemmstoffe der Cyclooxygenase I und/oder der Cyclooxygenase 2), Leukotrienantagonisten oder Hemmstoffe der Leukotrienbildung, Cytokinantagonisten oder Hemmstoffe der Cytokinbildung, Mastzellstabilisatoren (zum Beispiel Cromoglycinsäure), Antihistaminika (zum Beispiel Terfenadin), Cyclosporin A, FK506, antiinflammatorische Cytokine (zum Beispiel TGFβ, IL-10 etc.), Substanzen, die die Freisetzung von antiinflammatorischen Mediatoren veranlassen (zum Beispiel Cyclosporin A), antiinflammatorische Fettsäuren, deren Vorstufen oder Hemmstoffe des Abbaues der antiinflammatorischen Fettsäuren handeln. Als antiinflammatorische Therapieprinzipien im Rahmen der Kombinationstherapie sind auch Maßnahmen gemeint, die eine akute Entzündungsreaktion verursacht durch die Verabreichung des Antigens unterbinden. Hierzu zählen zum Beispiel die Ummantelung der Antigene mit Antikörpern oder Fragmenten von Antikörpern, galenische Maßnahmen, die eine akute Entzündungsreaktion vermeiden, jedoch eine ausreichende Antigenpräsentation erlauben. Zu den weiteren Maßnahmen zur Vermeidung von akut-entzündlichen Reaktionen, die den Aufbau einer regulatorischen Immunantwort negativ beeinflussen, gehören auch therapeutische Maßnahmen zur Bekämpfung und Vermeidung von Infektionen oder unspezifischen Entzündungsreaktionen. Hierzu zählt auch der Einsatz von Antibiotika, Chemotherapeutika, polyvalenten Immunglobulinen, Maßnahmen zur Vermeidung einer Gewebeischiämie, Virustatika und der Einsatz von Therapieprinzipien, die ganz allgemein Infektionen bekämpfen (auch chirurgische).

Die Gabe der Substanzen kann intravenös, inhalatorisch, subkutan, epikutan, rektal, intrathekal, transdermal oder über sonstige Applikationswege erfolgen.

Im allgemeinen hat es sich als vorteilhaft erwiesen, den Pyrimidinsynthese-inhibitor und das Antigen entweder 2 bis 3 Tage nach dem Verschwinden sämtlicher Symptome einer sich selbst begrenzenden Immunreaktion (zum Beispiel Infektion wie eine Bronchitis) oder einige Tage nach der gezielten Auslösung einer sich selbst begrenzenden Autoimmunreaktion (zum Beispiel durch anti-D-Serum bei Rhesus Faktor positiven Patienten ausgelöst) abzusetzen.

Infektionen, die das Immunsystem nicht erfolgreich bekämpfen kann, müssen medikamentös durch Antibiotika, Chemotherapeutika, polyvalente Immunglobuline, Immunstimulantien, chirurgische Eingriffe oder physikalische Maßnahmen eliminiert werden. Es muss auch eine Reinfektion mit den Problemkeimen durch medikamentöse Maßnahmen (zum Beispiel Antibiotika, Chemotherapeutika oder polyvalente Immunglobuline) oder physikalische Maßnahmen (zum Beispiel Expositionsprophylaxe durch Mundschutz) verhindert werden, da chronische Entzündungsreaktionen zu einem Zusammenbruch bestehender regulatorischer Immunreaktionen führen und somit zur Neuentstehung oder zum Wiederauftreten von unerwünschten schädlichen Immunreaktionen führen können.

Der Gegenstand der Erfindung wurde durch die folgenden Untersuchungen belegt: Die Untersuchungen wurden an freiwilligen Probanden durchgeführt, die an einer Kontaktdermatitis gegen ein oder mehrere Metalle oder an einer allergischen Reaktion gegen Gräserpollen litten.

Die Kontaktdermatitis wurde als Modellerkrankung für die Vielzahl der T-Zellvermittelten Immunreaktionen (allgemein: überschießende schädigende Immunreaktionen) ausgewählt, da sie durch Auftragen der Kontaktallergene auf die Haut einfach auszulösen ist und die Schwere der Entzündungs- und Immunreaktion durch Beobachtung der Hauterscheinungen einfach zu beurteilen ist.

Die Wirkung der Kombinationstherapie an der Typ I Allergie (allergische Konjunktivitis und Rhinitis) wurde untersucht, da es sich bei der Typ I Allergie um eine durch Antikörper verursachte Erkrankung handelt, die von T-Zellen reguliert wird. Bei der Rhinitis und Konjunktivitis konnten zusätzlich die Wirkstoffe Neomycin und Dexamethason lokal topisch verabreicht werden, da es sich bei den Oberflächen um Schleimhäute handelt, die die Resorption der Wirkstoffe sehr gut ermöglichen und die relevanten Bestandteile des Immunsystems (sekundäre lymphatische Organe) direkt in Kontakt mit der Schleimhaut stehen. Bei den Allergieuntersuchungen erfolgte der Kontakt zum Antigen aerogen durch Exposition zum Blütenstaub der Haselnuss bzw. zum Staub der Gräser.

Das erfindungsgemäße Kombinationspräparat kann auch Kompositionen oder Kombinationspackungen umfassen, in denen die Bestandteile nebeneinander enthalten sind und zur gleichzeitigen, getrennten oder zeitlich abgestuften Therapie von überschießenden, schädigenden Immunreaktionen und/oder degenerativen Prozessen an ein und denselben menschlichen oder tierischen Körper bereitgestellt werden. Bevorzugt ist die Applikation der Verbindung der Formel I und/oder II vor, gleichzeitig, zeitlich getrennt oder zeitlich abgestuft von der Applikation des Antigens (zum Beispiel Desmoglein 3) oder der Vorstufe des Antigens (zum Beispiel Vektor der die Produktion von Insulin oder Faktor VIII kodiert), welches bei den überschießenden schädigenden Immunreaktionen sowie degenerativen Prozessen (zum Beispiel Fragmente des Gelenkknorpels, spezifische Antigene von sekretorischen Gewebe, die im Alter degenerieren, wie zum Beispiel Testosteron produzierende Zellen) eine Rolle spielt. Hierzu wird beispielsweise zuerst N-(4-Trifluormethylphenyl)-2-cyan-3-hydroxy-crotonsäureamid appliziert. Zeitgleich wird zum Beispiel Desmoglein 3, ein Antigen, welches bei Pemphigus vulgaris eine zentrale Rolle spielt, subkutan oder intravenös verabreicht. Durch die Gabe von Desmoglein 3 wird eine Proliferation von Zellen des Immunsystems stimuliert, die sowohl steigernd als auch hemmend auf die Erkrankung Pemphigus vulgaris wirken. Durch die gleichzeitige oder zeitlich versetzte Gabe von N-(4-Trifluormethylphenyl)-2-cyan-3-hydroxy-crotonsäureamid wird bevorzugt die Vermehrung von Zellen des Immunsystems gehemmt, die die Autoimmunerkrankungen hervorrufen, während sich diejenigen Zellen, die die Autoimmunerkrankung unterdrücken, relativ unbeeinträchtigt vermehren. Dadurch kommt es nach einer einer gewissen Zeit der Gabe von Desmoglein 3 und N-(4-Trifluormethylphenyl)-2-cyan-3-hydroxy-crotonsäureamid zu einem Überwiegen der Aktivität von Zellen des Immunsystems, die die Autoimmunerkrankung Pemphigus vulgaris unterdrücken. Sobald dieser Zustand eingetreten ist, kann die Verabreichung der erfindungsgemäßen Kombinationspräparate beendet werden, ohne dass danach die Erkrankung wieder auftritt.

Die erfindungsgemäße Zubereitung kann als Dosiereinheit in Form von Arzneiformen wie Inhalationssystemen, Kapseln (einschließlich Mikrokapseln, die im allgemeinen keine pharmazeutischen Träger enthalten), Tabletten einschließlich Dragees und Pillen, oder Zäpfchen vorliegen, wobei bei Verwendung von Kapseln das Kapselmaterial die Funktion des Trägers wahrnimmt und der Inhalt zum Beispiel als Pulver, Gel, Lösung, Emulsion oder Dispersion vorliegen kann.

Besonders vorteilhaft und einfach ist es jedoch, orale (perorale) Formulierungen des Proteinsyntheseinhibitors/Nukleotidsyntheseinhibitors und des Antigens herzustellen, die die berechneten Mengen der Wirkstoffe zusammen mit dem gewünschten pharmazeutischen Träger enthalten. Insbesondere für das Antigen bzw.

Vorstufen des Antigens ist es vorteilhaft, Maßnahmen, wie zum Beispiel die Behandlung mit Polyethylenglykol durchzuführen, die die Resorption im Speziellen und den Transport zum Wirkort im Allgemeinen erleichtern und ermöglichen. Auch eine entsprechende Formulierung (Zäpfchen) für die rektale Therapie kann angewandt werden. Ebenso ist die transdermale/ epikutane / bucale / sklerale /nasale / pulmonale / intrathekale / okuläre / inhalative Applikation in Form von Salben, Cremes, Lösungen, Emulsionen und Pulvern möglich, die die erfindungsgemäße Zubereitung enthalten. Weiterhin ist die parenterale, intravenöse, intraarterielle, subkutane, intramuskuläre, intravesicale, intrathekale, okuläre, inhalative und intravaginale Applikation von Formulierungen möglich, die die erfindungsgemäße Zubereitung enthalten.

Salben, Pasten, Cremes und Puder können neben dem Wirkstoff die üblichen Trägerstoffe enthalten, z. B. tierische und pflanzliche Fette, Wachse, Paraffine, Stärke, Tragant, Cellulosederivate, Polyethylenglykole, Silicone, Kieselsäure, Aluminiumhydroxid, Talkum, Zinkoxid, Milchzucker, Bentonit, Calciumsilikat und Polyamidpulver oder Gemische dieser Stoffe. Die Tabletten, Pillen oder Granulatkörper können nach Verfahren wie Press-, Tauch- oder Wirbelbettverfahren oder Kesseldragierung hergestellt werden und enthalten Trägermittel und andere übliche Hilfsstoffe wie Gelatine, Agarose, Stärke (zum Beispiel Kartoffel-, Mais- oder Weizenstärke), Cellulose wie Ethylcellulose, Siliziumdioxid, Magnesiumcarbonat, verschiedene Zucker wie Milchzucker und/oder Calciumphosphate. Die Dragierlösung besteht gewöhnlich aus Zucker und/oder Stärkesirup und enthält meistens noch Gelatine, synthetische Celluloseester, Gummi arabicum, Polyvinylpyrrolidon, Pigmente, oberflächenäktive Substanzen, Weichmacher und ähnliche Zusätze entsprechend dem Stand der Technik. Zur Herstellung der Zubereitungsformen kann jedes übliche Fließregulierungs-, Schmier- oder Gleitmittel, wie Magnesiumstearat, und Trennmittel verwendet werden. Bevorzugt haben die Zubereitungen die Form von Mantel /Kern-Tabletten oder Mehrschichttabletten, wobei sich der Proteinsyntheseinhibitor / Nukleotidsyntheseinhibitor im Mantel bzw. im Kern bzw. in einer Schicht befindet, während sich das Antigen bzw. die Vorstufen des Antigens im Kern, im Mantel oder in einer anderen Schicht befindet. Die Wirkstoffkomponenten können auch in retardierter Form vorliegen oder an Retardierungsmaterial adsorbiert bzw. im Retardierungsmaterial (zum Beispiel Cellulose- oder Polystyrolharzbasis, zum Beispiel Hydroxyethylcellulose) eingeschlossen sein. Eine verzögerte Freisetzung der Wirkstoffe kann auch erreicht werden, indem die betreffende Schicht bzw. das Kompartiment mit üblichen magensaftunlöslichen Überzügen versehen wird.

Bevorzugt ist eine Applikation, bei der das Antigen bzw. Vorstufen des Antigens in die lokale Umgebung gelangen, in der die Immunreaktion, die zu behandeln ist, abläuft. Das Antigen kann aber auch systemisch verabreicht werden. Der Proteinsyntheseinhibitor/Nukleotidsyntheseinhibitor kann sowohl lokal oder auch systemisch wirksam verabreicht werden.

Die anzuwendende Dosierung ist selbstverständlich abhängig von verschiedenen Faktoren wie dem zu behandelnden Lebewesen (d. h. Mensch oder Tier), Alter, Gewicht, allgemeiner Gesundheitszustand, dem Schweregrad der Symptome, der zu behandelnden Erkrankung, eventuellen Begleiterkrankungen, (falls vorhanden) der Art der begleitenden Behandlung mit anderen Arzneimitteln oder der Häufigkeit der Behandlung. Die Dosierungen werden im allgemeinen mehrfach pro Tag und vorzugsweise einmal bis dreimal pro Tag verabreicht. Die verwendeten Mengen an Einzelwirkstoff orientieren sich hierbei an der empfohlenen Tagesdosis des jeweiligen Einzelwirkstoffs und sollen im allgemeinen im Kombinationspräparat bei 10% bis 300% der empfohlenen Tagesdosis liegen, bevorzugt bei 50% bis 150%, insbesondere bei 80%. Die geeignete Therapie mit den erfindungsgemäßen Kombinationen besteht somit zum Beispiel in der Verabreichung von einer, zwei oder drei Einzeldosierungen der Zubereitung bestehend aus N-(4-Trifluormethylphenyl)-5-methylisoxazol-4-carboxamid oder N-(4-Trifluormethylphenyl)-2-cyan-3-hydroxycrotonsäureamid in einer Menge von 2 mg bis 250 mg, bevorzugt 5 mg bis 150 mg, insbesondere 1 0 mg bis 50 mg, insbesondere bevorzugt 1 0 mg bis 20 mg und des Antigens in einer Menge von 1 00 mg bis 1 0000 mg, insbesondere von 1500 mg bis 3000 mg.

Ferner können die erfindungsgemäßen Zubereitungen auch zusammen mit anderen geeigneten Wirkstoffen, beispielsweise Antiuricopathika, Analgetika, steroidalen oder nichtsteroidalen Antiphlogistika, Thrombocytenaggregationshemmern, Cytokinen, Cytokinagonisten, Cytokinantagonisten oder immunsuppressiven Verbindungen wie zum Beispiel Cyclosporin A, FK 506 oder Rapamycin eingesetzt werden.

### Pathogenese der Kontaktdermatitis:

Während der Sensibilisierungsphase dringen die meist niedermolekularen Fremdsubstanzen über die Haut ein, werden direkt oder nach vorangegangener Bindung an Proteine von dendritischen Langerhans-Zellen aufgenommen, zu den regionalen Lymphknoten transportiert und dort HLA-Klasse II restringierten T-Lymphozyten präsentiert. Innerhalb weniger Tage führt deren Aktivierung zu einer expandierten, teils ortsständigen, teils im Organismus verteilten Kontaktallergen-spezifischen T-Zell-Subpopulation. Aufgrund der T-Zellabhängigkeit kann die Kontaktdermatitis als Modellerkrankung für alle überschießenden schädigenden Immunreaktionen gelten, die durch T-Zellen hervorgerufen werden und/oder durch mangelnde Eigenregulation des Immunsystems begründet sind.

### Auslösung der Kontaktdermatitis:

Die Kontaktallergien wurden durch Auftragen von Zubereitungen in weißer Vaseline ausgelöst. Diese Vaselinezubereitungen enthielten entweder 1% Kobalt-II-chlorid oder 5% Nickel-II-sulfat.

Zur Auslösung der Kontaktdermatitis wurde die Vaselinezubereitung einmal am Tag, meist am Ober- oder Unterarm auf einer Fläche von ca. 2 x 2 cm² aufgetragen.

Um den Juckreiz zu mildern konnten die Probanden nach eigenem Ermessen eine Lokalanästhetika haltige Salbe auf die entsprechenden Hautflächen auftragen (z.B. EMLA-Salbe).

### Krankheitsbild der Kontaktdermatitis und Einteilung entsprechend der Schwere der entzündlichen Reaktion:

Durch die Auftragung der Vaselinezubereitungen werden innerhalb einer Latenzzeit von mehreren Stunden bis zu zwei Tagen folgende Hautreaktionen ausgelöst:

Rötung, ödematöse Schwellung der Haut, Bläschen oder Blasenbildung, nässende Erosionen, Krustenbildung.

Entsprechend des Umfanges der entzündlichen Reaktion wurde die Kontaktdermatitis in folgende Stufen eingeteilt:
- Stufe 1:: sehr geringe Entzündungsreaktion, leichte Rötung der Haut;
- Stufe 2:: geringe Entzündungsreaktion, Rötung der Haut und eventueller Juckreiz;
- Stufe 3:: leichte Entzündungsreaktion, Rötung und ödematöse Schwellung der Haut, Juckreiz;
- Stufe 4:: mittlere Entzündungsreaktion, Rötung, ödematöse Schwellung, Bläschen oder Blasenbildung, Juckreiz;
- Stufe 5:: ausgeprägte Entzündungsreaktion: Rötung, ödematöse Schwellung, Bläschen und Blasenbildung, nässende Erosionen und eventuell Krustenbildung;

### Steuerung des Grades der entzündlichen Reaktion mittels Kortikoiden:

Durch die systemische Gabe von Ultralan® Tabletten wurde die Schwere der auftretenden Entzündungsreaktion gesteuert. Bei der Induktion der antigen-spezifischen regulatorischen Immunantwort wurde mittels Gabe von Fluocortolon (Ultralan®) die entzündliche Reaktion im Bereich der Stufe 1 und 2 gehalten. Entwickelte sich trotz der Gabe von Ultralan® eine Kontaktdermatitis der Stufe 3 oder höher, wurde die Dosis von Ultralan® auf die nächste Dosierungsstufe erhöht. Falls erforderlich, erfolgte eine weitere Erhöhung der Dosis 2 Tage nach der letzten Dosiserhöhung. In der Regel reichten Dosierungen von maximal 10 mg/Tag aus, um die Symptome der Kontaktallergie auf die Stufen 1 bis 2 zu beschränken.

Sobald keine Symptome der Kontaktdermatitis bei einer Dosierungsstufe von Ultralan® mehr festgestellt werden konnten, wurde die Dosis am nächsten Tag reduziert und anschließend wieder solange beibehalten, bis sämtliche Symptome der Kontaktallergie verschwunden waren, um dann anschließend wieder die Dosis an Ultralan zu reduzieren bis schließlich kein Ultralan® mehr verabreicht werden musste.

In der Regel wurden folgende Abstufungen der tägliche Dosierungen an Ultralan® eingesetzt: 20 mg, 15 mg, 10 mg, 7,5 mg, 5 mg, 2,5 mg, 0 mg;

### Anwendung von Leflunomid zum Aufbau der regulatorischen Immunantwort:

Ein entsprechender Blutspiegel an Leflunomid wurde durch eine Loadingdosis von 100mg an den ersten drei aufeinanderfolgenden Tagen der Leflunomidtherapie erzielt, anschließend wurde die Leflunomiddosis auf 20 mg pro Tag reduziert. Diese Dosis an Leflunomid wurde, falls nicht anders beschrieben, bis 30 Tage nach dem Zeitpunkt, an dem die Gabe von Flucortolon beendet wurde, beibehalten.

Anschließend wurde Leflunomid beschleunigt aus dem Körper durch 3 x tägliche Gabe von 8 Gramm Colestyramin über 10 Tage eliminiert.

### Verabreichung des Antigens während des Aufbaues der regulatorischen Immunantwort mittels Leflunomid bei der Kontaktdermatitis:

Wie oben beschrieben wird das Antigen 1 x täglich dermal über eine Zubereitung aus weißer Vaseline aufgetragen. Das Antigen wurde in der Regel bis zum Tag 10 nach der letzten Gabe von Leflunomid verabreicht.

### Provokationsuntersuchungen:

Bei den Untersuchungen zum Aufbau einer regulatorischen Immunantwort bei der Kontaktdermatitis, wurde eine erneute Exposition gegenüber dem Antigen, gegen das eine regulatorische Immunantwort aufgebaut wurde, frühestens 1 Monat nach dem letzten Kontakt mit dem Antigen, vorgenommen.

### Untersuchungsreihe 1:

### Untersuchung 1.1.

An 4 Probanden, die eine Kontaktallergie sowohl gegen Nickel als auch Kobalt aufwiesen, wurde zunächst jeweils eine Kontaktallergie gegen beide Allergene ausgelöst und anschließend die Dosis an Ultralan® ermittelt mit der sich die Kontaktallergie auf den Schweregrad 1 bis 2 einstellen ließ. Anschließend wurde die Dosis von Ultralan® nochmals für 3 Tage gerade soweit erhöht, dass keinerlei Hautreaktionen mehr beobachtet wurden. Dann wurde Ultralan® stufenweise jeden Tag bis zum kompletten Absetzen von Ultralan® reduziert. Bei allen Patienten trat die Kontaktdermatitis während der Reduzierung von Ultralan® wieder auf. Sobald sich eine Kontaktdermatitis des Schweregrades 3 entwickelt hat, wurde die Exposition zum Kontaktallergen beendet.

Anschließend wurde eine Pause von 1 Monat eingelegt, in der keinerlei Kontakt zu den entsprechenden Allergenen bestand und auch keinerlei das Immunsystem beeinflussende Medikamente gegeben wurden.

Nach einem Monat erhielten alle Probanden eine Loadingdosis von jeweils 100mg Leflunomid an 3 aufeinander folgenden Tagen. Anschließend erhielten die Probanden konstant eine tägliche Dosis von 20 mg Leflunomid.

Am 4. Tag der Leflunomidgabe wurde bei 2 Probanden eine Nickel haltige Vaseline und bei den beiden anderen Probanden die Kobalt haltige Vaseline zur Auslösung der Kontaktdermatitis aufgetragen.

Innerhalb von 2 Tagen entwickelte sich bei allen Probanden trotz der Gabe von Leflunomid eine Hautreaktion der Stufe 3 bis 4. Sofort nachdem die Hautreaktion die Stufe 3 erreicht hatte wurde mit der Gabe von Steroiden (Ultralan®) begonnen und die Kontaktallergie auf den Grad 1-2 eingestellt. Innerhalb weniger Tage (in der Regel 3 bis 5 Tage) verschwanden die Symptome der Kontaktallergie vollständig. Anschließend erfolgte, wie oben beschrieben, eine schrittweise Reduzierung der Steroiddosis unter Vermeidung einer akut entzündlichen Reaktion bis schließlich kein Steroid (Ultralan®) mehr gegeben wurde. Nach dem kompletten Absetzen der Steroide wurde während der nächsten 30 Tage täglich Leflunomid und das Antigen appliziert. Während dieser Phase waren weder bei den Probanden, die die Nickel haltige Vaseline, noch bei denjenigen Probanden, die die Kobalt haltige Vaseline erhielten, Symptome der Kontaktallergie zu beobachten. Nach 30 Tagen wurde auch die Verabreichung von Leflunomid beendet und die beschleunigte Elimination von Leflunomid mittels Colestyramin über 10 Tage vorgenommen. Während dieser beschleunigten Elimination erfolgte bei allen Probanden weiterhin die Exposition mit dem Kontaktallergen. Bei keinem der Probanden wurden während dieser Phase Symptome der Kontaktallergie beobachtet.

Nach diesem Zeitraum wurde auch die Exposition zum Kontaktallergen beendet.

4 Wochen nach Beendigung dieses Behandlungszykluses erfolgte eine erneute Exposition zu dem Antigen, welches während der Gabe von Leflunomid verabreicht wurde.

Bei keinem der Probanden traten innerhalb der 2 Wochen, in der sie mit dem Kontaktallergen provoziert wurden, Symptome einer Kontaktallergie auf.

### Untersuchung 1.2.

Anschließend wurde 4 Wochen gewartet, in denen die Probanden keinen Kontakt zu den Kontaktallergenen hatten.

Nach 4 Wochen wurden die Probanden demjenigen Antigen ausgesetzt, zu dem sie während der Leflunomidtherapie keinen Kontakt hatten.

Alle Probanden entwickelten innerhalb von 2 Tagen eine Kontaktdermatitis der Stufe 3 bis 4.

### Untersuchung 1.3.

Am Tag 3 wurde bei allen Probanden dasjenige Allergen abgesetzt, zu dem sie während der Leflunomidtherapie keinen Kontakt hatten; dafür wurde dasjenige Allergen aufgetragen, dem die Probanden während der Leflunomidtherapie ausgesetzt waren. Zu dem Zeitpunkt als mit der Gabe des zweiten Kontaktallergens begonnen wurde, zeigten die betroffenen Hautareale noch mittlere bis deutliche Entzündungsreaktionen. Überraschender Weise hielten die Symptome der Kontaktallergie solange an, wie das Kontaktallergen verabreicht wurde.

### Untersuchung 1.4.

Nach diesen Untersuchungen wurde wiederum eine Pause von 4 Wochen eingelegt. Nach diesen 4 Wochen wurden die Probanden demjenigen Antigen ausgesetzt, dem sie während der Leflunomidtherapie ausgesetzt waren und gegen das sie ca. 4 Wochen nach der Leflunomidtherapie nicht, jedoch ca. 4 Wochen vorher durch eine mittlere bis deutliche Entzündungsreaktion reagiert hatten.

Diesmal reagierten die Probanden mit keiner Kontaktallergie während des gesamten Expositionszeitraumes von 2 Wochen.

### Untersuchung 1.5.

Direkt nach diesen 2 Wochen wurde dasjenige Antigen verabreicht, dem die Probanden während der Leflunomidtherapie nicht ausgesetzt waren. Die Probanden entwickelten wieder innerhalb von 2 Tagen Symptome einer Kontaktallergie der Stufe 3 bis 4. Sobald die Entzündungsreaktion entwickelt war, wurde das Antigen abgesetzt und die Verabreichung desjenigen Antigens begonnen, das während der Leflunomid-gabe verabreicht wurde.

Wieder hielten die Symptome der Kontaktallergie über einen Zeitraum von 2 Wochen an. Nach diesem Zeitraum von 2 Wochen wurde das Kontaktallergen weiter verabreicht, jedoch wurde Ultralan® in einer Dosierung gegeben, sodass die Symptome der Kontaktallergie vollständig verschwanden. Sobald die Symptome der Kontaktallergie unter Ultralan® verschwunden waren, wurde die Gabe von Ultralan® täglich stufenweise reduziert und schließlich ganz abgesetzt. Obwohl auch nach dem Absetzen von Ultralan® das Allergen noch für 2 Wochen dermal verabreicht wurde, traten keine Symptome einer Kontaktallergie auf.

### Untersuchung 1.6.

4 Wochen später erfolgt nochmals eine Exposition gegen dasjenige Kontaktallergen, welches während der Leflunomidgabe verabreicht wurde. Während der ersten zwei Wochen der Exposition zu dem Allergen wurden keinerlei Symptome einer Kontakt-allergie beobachtet.

Nach diesen 2 Wochen erfolgte eine Bestrahlung der Hautfläche mit einer UV-A Lampe unter fortgeführter Exposition zu dem Kontaktallergen. Gleichzeitig hierzu erfolgt auf dem anderen Arm der Probanden eine identische Bestrahlung einer vergleichbaren Hautstelle, auf die Vaseline ohne Kontaktallergen aufgetragen wurde. Durch die UV-A Bestrahlung wurde eine leichte Entzündungsreaktion mit Rötung (kein Ödem) auf dem Arm verursacht, auf den kein Kontaktallergen aufgetragen wurde. Auf demjenigen Arm, auf den das Konataktallergen aufgetragen wurde, waren die Entzündungssymptome etwas stärker, obwohl exakt die gleichen Strahlungsbedingungen und Expositionzeiten für beide Arme gewählt wurden.

An dem Arm, auf den kein Kontaktallergen aufgetragen wurde, verschwand die Rötung spätestens nach 4 Tagen. Demgegenüber waren die Entzündungsreaktionen auf derjenigen Seite, auf der das Kontaktallergen aufgetragen wurde, frühestens nach 7 Tagen verschwunden.

### Untersuchungsreihe 2:

### Untersuchung 2.1.

Im Versuch 2 wurden bei 2 Probanden genauso wie im Versuch 1 versucht, eine regulatorische Immunantwort aufzubauen.

Im Gegensatz zu Versuch 1 wurde jedoch eine Dosis an Ultralan® gewählt, bei der keinerlei Symptome der Kontaktallergie mehr beobachtet wurden. Eine Reduzierung der Ultralan® Verabreichung erfolgte während der ersten 4 Wochen nicht. Nach 4 Wochen wurde Ultralan® komplett abgesetzt und versucht, weiterhin Leflunomid und das Kontaktallergen zu verabreichen. Bereits einen Tag nach dem Absetzen von Ultralan entwickelten sich massiv Symptome der Kontaktallergie, sodaß keine weiteren Untersuchungen vorgenommen wurden.

### Untersuchung 2.2.

4 Wochen später wurde begonnen, diese Probanden entsprechend der Kombinationstherapie aus Versuch 1 zu behandeln. Bei beiden Probanden konnte durch das Behandlungsschema aus Versuch 1 eine regulatorische Immunantwort aufgebaut werden, die auch bei anschließender Antigenexposition verhinderte, dass Symptome einer Kontaktallergie auftraten.

### Untersuchungsreihe 3:

### Untersuchung 3.1.

Im Versuch 3 wurde bei 2 weiteren Probanden mit Kontaktallergie der Versuch des Aufbaues einer regulatorischen Immunantwort entsprechend dem Behandlungsschema von Versuch 1 vorgenommen. Im Gegensatz von Versuch 1 wurde jedoch die Behandlungphase mit Kontaktallergen und Leflunomid ohne Steroide von 30 Tagen auf 2 Wochen verkürzt.

4 Wochen nach dem Absetzen von Leflunomid erfolgte eine Exposition zu dem Allergen. Drei bis vier Tage nach Beginn der Antigenexposition entwickelte sich zuerst eine leichte Rötung mit in den folgenden Tagen zunehmenden Symptomen einer Kontaktallergie.

### Untersuchung 3.2.

Durch eine einmalige Gabe von 5 mg Ultralan® und der gleichzeitigen Gabe von Leflunomid verschwanden die Symptome innerhalb eines Tages.

### Untersuchung 3.3.

Die Gabe von Leflunomid und des Kontaktallergens wurden anschließend über 20 Tage fortgesetzt, um dann wie im Versuch 1 Leflunomid beschleunigt zu eliminieren und die Gabe des Kontaktallergens zu beenden.

4 Wochen danach konnte wie in Versuch 1 gezeigt werden, daß sich eine stabile antigen-spezifische regulatorische Immunantwort aufgebaut hat.

### Untersuchungsreihe 4:

### Untersuchung 4.1.

Bei Untersuchungsreihe 4 wurde bei 2 Patienten, bei denen eine allergische Rhinitis/Conjunktivitis gegen zwei unterschiedliche Antigene (Haselnusspollen und Gräserpollen) vorlag, eine antigen-spezifische regulatorische Immunantwort mit Hilfe von Leflunomid selektiv gegen eines der beiden Antigene aufgebaut.

Zunächst wurden die Probanden dem Staub der Allergene ausgesetzt, um das Bestehen der Allergie zu belegen. Anschließend wurde eine Pause von 1 Monat eingelegt, in der die Probanden keinerlei Kontakt zu den entsprechenden Allergenen hatten und auch keinerlei das Immunsystem beeinflussende Medikamente gegeben wurden.

Nach einem Monat erhielten beide Probanden eine Loadingdosis von jeweils 100mg Leflunomid an 3 aufeinander folgenden Tagen. Anschließend erhielten die Probanden konstant eine tägliche Dosis von 20 mg Leflunomid.

Am 4. Tag der Leflunomidgabe wurden die Probanden einem der beiden Allergene ausgesetzt. Die Probanden reagierten sofort mit allergischen Symptomen. Die Symptome der Allergie wurde durch Gabe von 10 mg Ultralan® und von Vasokonstringentien (Otriven® Nasentropfen und Yxin® Augentropfen) soweit unterdrückt, daß nur noch leichte Symptome einer allergischen Reaktion, wie schwacher Juckreiz und leicht erhöhter Nasensekretfluss unmittelbar nach der Antigenexposition auftraten.

Innerhalb von 4 Tagen verschwanden die Symptome der Allergie direkt nach der Antigenexposition vollständig unter konstanter Ultralan® und Leflunomid Therapie. Anschließend erfolgte eine schrittweise Reduzierung der Steroiddosis unter Beibehaltung der Leflunomiddosis und Vermeidung einer akut entzündlichen Reaktion, bis schließlich kein Steroid (Ultralan®) mehr gegeben wurde. Nach dem kompletten Absetzen der Steroide wurde während der nächsten 30 Tage täglich Leflunomid und das Antigen verabreicht.

Nach diesen 30 Tagen wurde das Leflunomid beschleunigt durch 3 x tägliche Gabe von Colestyramin über 10 Tage aus dem Körper eliminiert. Während dieser beschleunigten Elimination wurde die tägliche Antigenexposition fortgesetzt.

Bei beiden Probanden traten hierbei keine Symptome einer Allergie auf.

Nach diesem Behandlungszyklus wurde ein Monat Ruhephase eingelegt, in dem die Probanden keine Medikamente zur Behandlung der Allergie erhielten.

Nach einem Monat wurden die Probanden dem Allergen (Blütenstaub) ausgesetzt, dem sie während des Behandlungszyklus mit Leflunomid ausgesetzt waren. Trotz der fortgesetzten täglichen Allergen Exposition über 2 Wochen entwickelten die Probanden keinerlei Symptome einer allergischen Reaktion.

### Untersuchung 4.2.

4 Wochen später, in denen die Probanden keinen Kontakt zu den Allergenen hatten, wurden sie demjenigen Antigen (1 x täglich Staub der Gräserpollen) ausgesetzt, zu dem sie während der Therapie mit Leflunomid keinen Kontakt hatten.

Beide Probanden entwickelten innerhalb von 2 Tagen Symptome einer Allergie.

### Untersuchung 4.3.

Sobald die Probanden Symptome einer Allergie mit den Symptomen einer Entzündungsreaktion aufwiesen, wurden sie für 2 weitere Tage sowohl dem Allergen ausgesetzt, welches in Kombination mit Leflunomid gegeben wurde, als auch dem Allergen, das nicht in Kombination mit Leflunomid gegeben wurde. Am 3. Tag und 4. Tag wurde nur noch das Antigen verabreicht, das ursprünglich in Kombination mit Leflunomid gegeben wurde. Sowohl am 3. als auch am 4. Tag entwickelten sich Symptome einer Allergie. In den folgenden beiden Tagen wurde vor der Exposition des Antigens Dexamethason Nasenspray und Augentropfen verabreicht, damit sich keine Entzündungsreaktionen entwickeln konnten. Diese Maßnahmen dienten auch dazu, die Antikörper, die sich über einen längeren Zeitraum auf der Oberfläche von Zellen des Immunsystems befinden können, zu verbrauchen.

### Untersuchung 4.4.

Nach wiederum 4 Wochen wurden die Probanden nochmals dem Antigen ausgesetzt, dem sie während der Leflunomid Verabreichung ausgesetzt waren. Selbst nach 2 Wochen der täglichen Exposition mit dem Antigen haben sich keine Symptome einer Allergie entwickelt.

### Untersuchungsreihe 5:

### Untersuchung 5.1.

Bei Untersuchungsreihe 5 wurde wie bei Untersuchungsreihe 4 bei 2 Patienten, bei denen eine allergische Rhinitis/Conjunktivitis gegen zwei unterschiedliche Antigene (Haselnusspollen und Gräserpollen) vorlag, eine antigen-spezifische regulatorische Immunantwort gegen eines der beiden Antigene aufgebaut.

Während der Allergenexposition wurde mittels Dexamethason haltiger Nasensprays und Augentropfen, die Immunreaktion soweit reduziert, dass keine akuten Entzündungssymptome mehr beobachtet wurden. Zusätzlich konnten die Probanden Vasokonstringentien wie Otriven® Nasentropfen und Yxin® Augentropfen zur Linderung akuter Symptome einsetzen. Eine leicht vermehrte Bildung von Nasensekret und leichter Juckreiz der Augen unter Allergenexposition konnten allerdings auftreten.

Als immunmodulatorische Substanz wurde Neomycinsulfat in einer Konzentration von 50mg/ml in physiologischer Kochsalzlösung als Augentropfen und als Dosiernasenspray eingesetzt. Während der Allergenexposition (Pollenflugsaison) wurde tagsüber zunächst alle 3 Stunden die Neomycinlösung verabreicht. Sobald Symptome einer allergischen Reaktion wie starker Juckreiz, Augentränen, Fluss von Nasensekret oder Niesreiz auftraten, wurden diese Entzündungsreaktion innerhalb der ersten 5 Tage durch Einsatz der Dexamethasonaugentropfen, des Dexamethasonnasensprays, von Xylometazolinnasentropfen und Yxin® Augentropfen unterdrückt. Die Verabreichung der Steroide und der Vasokonstringentien erfolgte nach Bedarf.

Innerhalb der ersten 5 Tage reduzierte sich der Bedarf an Dexamethasongaben von einer durchschnittlich 4 x täglichen Gabe auf eine 2 x tägliche Gabe am Morgen und am Nachmittag.

In den folgenden 4 Tagen wurde anstelle von Dexamthason Cromoglycinsäure verabreicht, gleichzeitig konnte die Verabreichung von Neomycinlösung auf morgens, mittags und abends reduziert werden. Nach diesen 4 Tagen wurde keine Cromoglycinsäure mehr benötigt. Die Gabe der Neomycinlösung wurde für zwei weitere Woche unverändert fortgeführt. Nach einer Woche wurden die Probanden zweimal täglich dem Antigen verstärkt ausgesetzt (direkte Exposition zu Blütenstaub der Haselnuss). Bei beiden Probanden traten hierbei keine Symptome einer Allergie auf.

Nach diesem Behandlungszyklus wurde ein Monat Ruhephase eingelegt, in der die Probanden keine Medikamente zur Behandlung der Allergie erhielten.

Nach einem Monat wurden die Probanden dem Allergen (Blütenstaub) ausgesetzt, dem sie während des Behandlungszyklus mit Neomycin ausgesetzt waren.
Die Probanden zeigten keine Symptome einer Allergie während dieser 2 Wochen andauernden täglichen Allergen Exposition.

### Untersuchung 5.2.

4 Wochen später in denen die Probanden keinen Kontakt zu den Allergenen hatten, wurden sie demjenigen Antigen (1 x täglich Staub der Gräserpollen) ausgesetzt, zu dem sie während der Therapie mit Neomycin keinen Kontakt hatten.

Beide Probanden entwickelten innerhalb von 2 Tagen Symptome einer Allergie.

### Untersuchung 5.3.

Sobald die Probanden Symptome einer Allergie mit den Symptomen einer Entzündungsreaktion aufwiesen, wurden sie für 2 weitere Tage sowohl dem Allergen ausgesetzt, welches in Kombination mit Neomycin gegeben wurde als auch demjenigen Antigen, das nicht in Kombination mit Neomycin gegeben wurde. Am 3. Tag und 4. Tag wurde nur das Antigen verabreicht, das ursprünglich in Kombination mit Neomycin gegeben wurde. Sowohl am 3. als auch am 4. Tag entwickelten sich Symptome einer Allergie. In den folgenden beiden Tage wurde vor der Exposition des Antigens Dexamethason verabreicht, damit sich keine Entzündungsreaktionen entwickeln konnten. Diese Maßnahmen dienten auch dazu, die Antikörper, die sich über einer längeren Zeit auf der Oberfläche von Zellen des Immunsystems befinden, zu verbrauchen.

### Untersuchung 5.4.

Nach wiederum einer Pause von 4 Wochen wurden die Probanden nochmals dem Antigen ausgesetzt, dem sie während der Neomycingabe ausgesetzt waren. Selbst nach 2 Wochen der täglichen Exposition mit dem Antigen hatten sich keine Symptome einer Allergie entwickelt.

### Aussagen der Untersuchungen:

Untersuchungsreihe 1.1. zeigt, dass sich durch eine Kombinationstherapie bestehend aus Leflunomid und dem auslösenden Allergen eine bleibende Nicht-Reaktivität gegenüber dem verabreichten Antigen hervorrufen läßt. Diese Nicht-Reaktivität wurde bei einer bereits aktiven Immunreaktion aufgebaut. Es wurde ebenfalls gezeigt, dass sich die Nicht-Reaktivität des Immunsystems nicht alleine durch stufenweise Reduzierung der Gabe von Ultralan® erzielen lässt. Die Untersuchung 1.2. belegt, dass diese Nicht-Reaktivität nur gegenüber dem während der Leflunomidtherapie verabreichten Antigen besteht und das Immunsystem nicht unspezifisch in seiner Reaktionsfähigkeit geschädigt wurde. Die Untersuchungen 1.2. beweisen, dass durch die Kombinationstherapie eine antigen-spezifische Nicht-Reaktivität hervorgerufen wurde.

Untersuchung 1.3. belegt, dass die antigen-spezifische Nicht-Reaktivität unter entzündlichen Bedingungen verloren geht. Daraus kann geschlossen werden, daß durch die Kombinationstherapie von Leflunomid mit einem Antigen dem Immunsystem keine Reaktionsmöglichkeiten verloren gehen, sondern dass diese im Rahmen von Entzündungsreaktionen wieder zur Verfügung stehen. Daher belegt Untersuchung 1.3. auch, dass durch eine Kombinationstherapie des Antigens mit Leflunomid eine antigen-spezifische regulatorische Immunantwort aufgebaut wurde. Untersuchung 1.4. belegt, dass sich nach Rückbildung der Entzündungsreaktion die antigen-spezifische Nicht-Reaktivität des Immunsystems ohne weitere therapeutische Maßnahmen wieder einstellt. Untersuchung 1.5 belegt, dass sich die antigen-spezifische Nicht-Reaktivität auch dann wieder einstellt, wenn die Entzündungsreaktion mit Hilfe von Medikamenten kurzfristig unterdrückt wird.

Untersuchung 1.6. belegt, dass der Zusammenbruch der antigen-spezifischen Nicht-Reaktivität nicht nur durch immunologisch ausgelöste Entzündungsreaktionen, sondern ganz allgemein durch unspezifische Entzündungsreaktionen hervorgerufen werden kann. Weiterhin belegt diese Untersuchung, dass sich bei leichten Entzündungsreaktionen trotz Kontaktes zum Antigen nach dem Wegfall der Ursache der Entzündungsreaktion die Nicht-Reaktivität selbstständig wiederaufbaut.

Untersuchungsreihe 2.1. belegt, dass während des Aufbaus der antigen-spezifischen regulatorischen Immunantwort die Immunreaktion nicht vollständig unterdrückt werden darf. Untersuchung 2.2. belegt, dass es sich bei den in 2.1. behandelten Probanden nicht um therapieresistente Fälle handelt.

Untersuchungsreihe 3 belegt, dass auch nach dem vollständigen Absetzen der Steroidtherapie alleine unter der gleichzeitigen Gabe von Leflunomid und dem Antigen sich die antigen-spezifische regulatorische Immunantwort in zeitlicher Abhängigkeit weiter aufbaut. Diese Untersuchungen belegen auch, dass durch die alleinige Gabe von Leflunomid während einer Immunreaktion keine ausreichend stabile antigen-spezifische regulatorische Immunantwort aufgebaut wird. Sie belegen, dass auch nach dem Verschwinden sämtlicher Symptome der Erkrankung weiterhin noch Antigen in Kombination mit Leflunomid verabreicht werden muss, damit sich eine ausreichend stabile antigen-spezifische regulatorische Immunreaktion etabliert, die auch nach dem Absetzten von Leflunomid das Auftreten der unerwünschten überschießenden schädlichen Immunreaktionen verhindert.

In der Untersuchungsreihe 4 wurde gezeigt, dass der Aufbau einer antigen spezifischen regulatorischen Immunantwort mit Hilfe der Kombinationstherapie von Leflunomid und einem Antigen auch bei Antikörper vermittelten Immunreaktionen möglich ist.

In der Untersuchungsreihe 5 wurde belegt, dass unter dem zeitlich begrenzten Einsatz von Neomycinaugentropfen und eines Neomycinnasensprays unter Vermeidung von akuten Entzündungsreaktionen und unter Kontakt zum betreffenden Antigen, eine antigen-spezifische regulatorische Immunantwort aufgebaut werden kann. Wie bei der regulatorischen Immunantwort, die mit Hilfe von Leflunomid aufgebaut wurde, zeigt auch die mit Hilfe von Neomycin aufgebaute regulatorische Immunantwort, dass die Nicht-Reaktivität des Immunsystems durch unspezifische Entzündungsreaktionen zusammenbrechen kann, sich aber nach dem Verschwinden der unspezifischen Entzündungsursache selbstständig wieder aufbaut.

## Patentansprüche

1. Pharmazeutisches Kombinationspräparat, **dadurch gekennzeichnet, dass** es enthält
a) mindestens einen Pyrimidinsynthese-Inhibitor aus der Gruppe Brequinar, Verbindung der Formeln I oder II 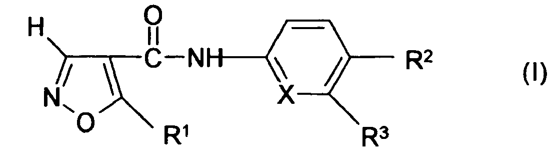 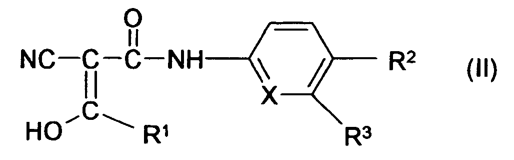 stereoisomene Formen von Verbindungen der Formel (I) oder (II) und ein physiologisch verträgliches Salz der Verbindung der Formel (II), in der die Substituenten folgende Bedeutung haben:
R¹ ist
a) -(C₁-C₄)-Alkyl,
b) -(C₃-C₅)-Cycloalkyl,
c) -(C₂-C₆)-Alkenyl, oder
d) -(C₂-C₆)-Alkynyl;
R² ist
a) -CF₃,
b) -O-CF₃,
c) -S-CF₃,
d) -OH,
e) -NO₂,
f) Halogen,
g) Benzyl,
h) Phenyl,
i) -O-phenyl, welches unsubstituiert ist,
j) -CN, oder
k) -O-phenyl, das mono- oder polysubstituiert ist durch eine Gruppe ausgewählt aus
1) (C₁-C₄)-Alkyl,
2) Halogen,
3) -O-CF₃ und
4) -O-CH₃;
R³ ist
a) -(C₁-C₄)-Alkyl,
b) Halogen, oder
c) ein Wasserstoffatom; oder
X ist
a) eine CH-Gruppe
b) ein Stickstoffatom.
oder eine Mischung von Brequinar und Verbindungen der Formel (I) und (II) oder von Salzen der Verbindungen der Formel (II)und
b) mindestens ein Antigen aus der Gruppe natürliche oder künstlich veränderte Zellverbände, Zellen, Zellfragmente, Proteine, Proteinfragmente, Vorstufen von Antigenen oder andere Verbindungen, die antigen wirksam sind.

2. Kombinationspräparat nach Anspruch 1, **dadurch gekennzeichnet, dass** es als Nukleotidsynthese-Inhibitor das N-(4-Trifluoromethylphenyl)-5-methylisoxazol-4-carboxamid der Formel (I) oder N-(4-Trifluoromethylphenyl)-2-cyano-5-hydroxycrotonamid, 2-Cyano-3-cyclopropyl-3-hydroxyacrylsäure (4-cyanophenyl)amid oder N-(4-Trifluoromethylphenyl)-2-cyano-3-hydroxyhept-2-en-6-yncarboxamid als Verbindungen der Formel (II) enthält.

3. Kombinationspräparat nach den Ansprüchen 1 oder 2, **dadurch gekennzeichnet, dass** es als einen eine akute entzündliche Reaktion unterdrückenden Wirkstoff ein Steroid, ein nicht-steroidales Antiphlogistikum, einen Leukotrienantagonisten, einen Cytokinantagonisten, einen Mastzellstabilisator, ein Antihistaminikum, ein Cyclosporin, FK 506, ein antiinflammatorisches Cytokin oder eine antiinflammatorische Fettsäure oder deren Vorstufen oder Hemmstoffe enthält.

4. Kombinationspräparat nach den Ansprüchen 1 bis 3, **dadurch gekennzeichnet, dass** in ihm die einzelnen Wirkstoffe zur gleichzeitigen, getrennten oder zeitlich abgestuften Therapie von überschießenden, schädigenden Immunreaktionen und/oder degenerativen Prozessen bereitgestellt werden.

5. Kombinationspräparat nach den Ansprüchen 1 bis 4, **dadurch gekennzeichnet, dass** es wenigstens einen weiteren Wirkstoff, einen galenischen Hilfsstoff und/oder einen Trägerstoff enthält.

6. Verwendung des Kombinationspräparates nach den Ansprüchen 1 bis 5 zur Herstellung eines Arzneimittels zur Therapie von überschießenden, schädigenden Immunreaktionen und/oder degenerativen Prozessen.

7. Verwendung gemäß Anspruch 6, **dadurch gekennzeichnet, dass** es sich um eine allergische Reaktionen, Transplantatabstoßungen oder Immunreaktionen gegen gentechnisch veränderte Zellen oder deren Produkte wie Faktor VIII oder Insulin handelt oder eine Autoimmunerkrankungen wie Multiple Sklerose, rheumatoide Arthritis, Pemphigus vulgaris und Thyreoiditis Hashimoto oder eine Erkrankung wie Colitis ulcerosa, Morbus Crohn, Psoriasis oder Arteriosklerose oder Arthrose, Demenserkrankungen oder Colitis ulcerosa.

## Claims

1. A pharmaceutical combination preparation, which comprises
a) at least one pyrimidine synthesis inhibitor from the group brequinar, a compound of the formula I or II 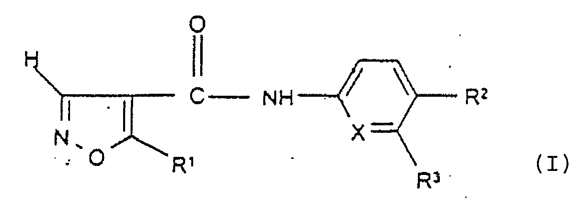 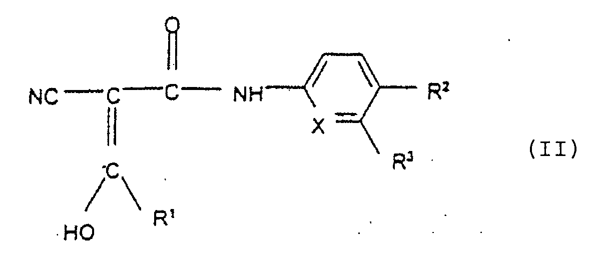 stereoisomeric forms of compounds of formula (I) or (II) and a physiologically tolerated salt of the compound of the formula (II), in which the substituents have the following meanings:
R¹ is
a) - (C₁-C₄) -alkyl,
b) - (C₃-C₅) -cycloalkyl,
c) - (C₂-C₆) -alkenyl, or
d) - (C₂-C₆) -alkynyl;
R² is
a) -CF₃,
b) -O-CF₃,
c) -S-CF₃,
d) -OH,
e) -NO₂,
f) halogen,
g) benzyl,
h) phenyl,
i) -O-phenyl which is unsubstituted,
j) -CN, or
k) -O-phenyl which is monosubstituted or polysubstituted by a group selected from
1) (C₁-C₄) -alkyl,
2) halogen,
3) -O-CF₃ and
4) -O-CH₃;
R³ is
a) - (C₁-C₄)-alkyl,
b) halogen, or
c) a hydrogen atom; or
X is
a) a CH group,
b) a nitrogen atom,
or a mixture of brequinar and compounds of the formula (I) and (II) or of salts of the compounds of the formula (II) and
b) at least one antigen from the group natural or artificially altered cell formations, cells, cell fragments, proteins, protein fragments, precursors of antigens or other compounds which are active antigenically.

2. The combination preparation as claimed in claim 1, which comprises, as nucleotide synthesis inhibitor, the N-(4-trifluoromethylphenyl)-5-methyl-isoxazole-4-carboxamide of the formula (I) or N-(4-trifluoromethylphenyl)-2-cyano-5-hydroxycrotonamide, 2-cyano-3-cyclopropyl-3-hydroxyacrylic acid (4-cyanophenyl)amide or N-(4-trifluoromethylphenyl)-2-cyano-3-hydroxyhept-2-en-6-ynecarboxamide, as compounds of the formula (II).

3. The combination preparation as claimed in claims 1 or 2, which comprises, as an active compound which suppresses an acute inflammatory reaction, a steroid, a nonsteroidal anti-inflammatory agent, a leukotriene antagonist, a cytokine antagonist, a mast cell stabilizer, an antihistamine, a cyclosporin, FK 506, an anti-inflammatory cytokine or an anti-inflammatory fatty acid, or their precursors or inhibitors.

4. The combination preparation as claimed in claims 1 to 3, wherein, in this preparation, the individual active compounds are provided for the simultaneous, separate or chronologically staggered treatment of overshooting, damaging immune reactions and/or degenerative processes.

5. The combination preparation as claimed in claims 1 to 4, which comprises at least one further active compound, a galenic auxiliary substance and/or a carrier substance.

6. The use of the combination preparation as claimed in claims 1 to 5 to produce a drug for treating overshooting, damaging immune reactions and/or degenerative processes.

7. The use as claimed in claim 6, wherein the immune reactions and/or degenerative processes are allergic reactions, transplant rejections or immune reactions against genetically altered cells or their products, such as factor VIII or insulin, or autoimmune diseases such as multiple sclerosis, rheumatoid arthritis, pemphigus vulgaris and Hashimoto's thyroiditis or a disease such as ulcerative colitis, Crohn's disease, psoriasis or arteriosclerosis or arthrosis, dementia diseases or ulcerative colitis.

## Revendications

1. Association médicamenteuse, **caractérisée en ce qu'**elle contient
a) au moins un inhibiteur de synthèse de pyrimidine du groupe du brequinar, de composés des formules I ou II : 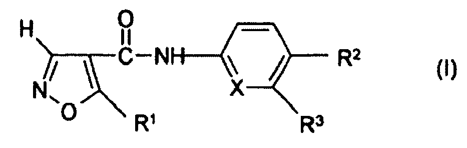 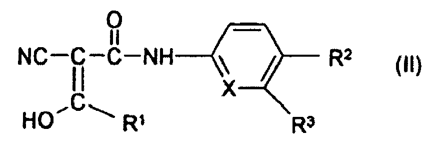 de formes stéréoisomères des composés des formules (I) ou (II) et d'un sel physiologiquement compatible du composé de la formule (II), où les substituants ont la signification suivante :
R¹ est un groupe
a) alkyle en C₁-C₄,
b) cycloalkyle en C₃-C₅,
c) alcényle en C₂-C₆, ou
d) alcynyle en C₂-C₆,
R² représente
a) -CF₃,
b) -O-CF₃,
c) -S-CF₃,
d) -OH,
e) -NO₂,
f) de l'halogène,
g) du benzyle,
h) du phényle,
i) un groupe -O-phényle qui n'est pas substitué,
j) -CN, ou
k) un groupe -O-phényle, qui est monosubstitué ou polysubstitué par un groupe choisi parmi
1) un groupe alkyle en C₁-C₄,
2) de l'halogène,
3) -O-CF₃ et
4) -O-CH₃,
R³ est
a) un groupe alkyle en C₁-C₄,
b) de l'halogène, ou
c) un atome d'hydrogène, ou
X est
a) un groupe CH
b) un atome d'azote, ou un mélange de brequinar et de composés des formules (I) et (II) ou de sels des composés de la formule (II) et
b) au moins un antigène du groupe des assemblages cellulaires naturels ou modifiés de manière artificielle, des cellules, des fragments de cellule, des protéines, des fragments de protéine, des précurseurs d'antigènes ou d'autres composés qui ont une action antigénique.

2. Préparation de combinaison suivant la revendication 1, **caractérisée en ce que**, comme inhibiteur de synthèse de nucléotide, elle contient le N-(4-trifluorométhylphényl)-5-méthylisoxazol-4-carboxamide de la formule (I) ou du N-(4-trifluorométhylphényl)-2-cyano-5-hydroxycrotonamide, du (4-cyano-phényl)amide d'acide 2-cyano-3-cyclopropyl-3-hydroxyacrylique ou du N-(4-trifluorométhylphényl)-2-cyano-3-hydroxyhept-2-èn-yncarboxamide comme composés de la formule (II).

3. Préparation de combinaison suivant les revendications 1 ou 2, **caractérisée en ce que**, comme substance active supprimant une réaction inflammatoire aiguë, elle contient un stéroïde, un antiphlogistique non stéroïdique, un antagoniste de leucotriène, un antagoniste de cytokine, un stabilisant de mastocyte, un antihistaminique, une cyclosporine, FK 506, une cytokine anti-inflammatoire ou un acide gras anti-inflammatoire ou leurs précurseurs ou des substances inhibitrices.

4. Préparation de combinaison suivant les revendications 1 à 3, **caractérisée en ce qu'**en elle les différentes substances actives sont tenues prêtes pour une thérapie simultanée, séparée ou étagée dans le temps de réactions immunitaires dommageables, excessives et/ou de processus de dégénération.

5. Préparation de combinaison suivant les revendications 1 à 4, **caractérisée en ce qu'**elle contient au moins une autre substance active, un adjuvant galénique et/ou une matière de support.

6. Utilisation de la préparation de combinaison suivant les revendications 1 à 5, pour la fabrication d'un médicament destiné à la thérapie de réactions immunitaires dommageables, excessives et/ou de processus de dégénération.

7. Utilisation suivant la revendication 6, **caractérisée en ce qu'**il s'agit de réactions allergiques, de rejets de greffes ou de réactions immunitaires contre des cellules génétiquement modifiées ou leurs produits, comme du facteur VIII ou de l'insuline, ou de maladies auto-immunitaires, comme la sclérose multiple, l'arthrite rhumatoïde, le pemphigus simple et la thyroïdite de Hashimoto ou d'une maladie, telle que la colite ulcéreuse, la maladie de Crohn, le psoriasis ou l'artériosclérose ou l'arthrose, des maladies de démence ou la colite ulcéreuse.
